# NEUE EUROPÄISCHE PATENTSCHRIFT

(11) **EP 0 577 636 B2**
(45) Veröffentlichungstag und Bekanntmachung des Hinweises auf die Entscheidung über den Einspruch: **24.01.2001**
(45) Hinweis auf die Patenterteilung: 14.12.1994
(21) Anmeldenummer: 92906596.9
(22) Anmeldetag: 18.03.1992
(51) Int. Cl.: A61K 7/06

(54) **HAARKOSMETISCHE ZUBEREITUNGEN**
COSMETIC PREPARATIONS FOR THE HAIR
COMPOSITIONS COSMETIQUES POUR LES CHEVEUX

(30) Priorität: 27.03.1991 DE 4109999
(43) Veröffentlichungstag der Anmeldung: 12.01.1994
(73) Patentinhaber: Henkel Kommanditgesellschaft auf Aktien, 40191 Düsseldorf (DE)
(72) Erfinder: GIEDE, Karl, D-4010 Hilden (DE); SEIDEL, Kurt, D-4000 Düsseldorf 13 (DE); MÜLLER, Reinhard, D-5140 Erkelenz 7 (DE); HOLLENBERG, Detlef, D-4006 Erkrath (DE)
(86) Internationale Anmeldenummer: EP9200586
(87) Internationale Veröffentlichungsnummer: WO9217153

(56) Entgegenhaltungen:
- EP-A- 0 052 489
- EP-A- 0 395 332
- WO-A-87/03196
- FR-A- 2 665 544
- GB-A- 2 113 245
- GB-A- 2 148 714
- GB-A- 2 188 948
- US-A- 4 269 824
- US-A- 4 690 818
- Patent Abstracts of Japan, Vol. 12, No. 275 (C-516)(3122) 29 June 1988,
- Patent Abstracts of Japan,Vol. 12, No. 28 (C-471)(2875) 27 January 1988
- SEIFEN OELE FETTE WASCHE, Vol. 113, No. 17, October 1987, Augsburg, DE, pages 617-622 "QUATERNISED PROTEINS IN MODERN HAIR CARE", CHESTER et al.
- E.S.Stern,V.L.Johnson,Cosmetic&Toiletries,vol.98(May83),S.76-78,80-82,84;
- H.P.Fiedler,Lexikon der Hilfsstoffe für Pharmazie,Kosmetik und angrenzende Gebiete,3.Auflage S.150,737;
- Grundlagen und Rezepturen der Kosmetika, 2.Auflage, Seiten 722-723,728-737.

## Beschreibung

Die Erfindung betrifft haarkosmetische Zubereitungen, die eine spezielle Wirkstoffkombination, wie in den Ansprüchen definiert, enthalten.

Das menschliche Haupthaar wird heute in vielfältiger Weise mit haarkosmetischen Zubereitungen behandelt. Dazu gehört beispielsweise die Reinigung der Haare mit Shampoos und Duschpräparaten und das Bleichen, Färben und Verformen von Haaren mit Wellmitteln, Tönungsmitteln und Stylingpräparaten. Als Folge fast aller Haarbehandlungen kann es zu unerwünschten Beeinträchtigungen der Haarstruktur kommen. Diese Beeinträchtigungen zeigen sich u.a. in einer schlechten Naß- und Trockenkämmbarkeit, einer verstärkten elektrostatischen Aufladung, verstärkter Sprödigkeit, verringerter Höchstreißkraft und Reißdehnung der Haare sowie einem verschlechterten äußeren Erscheinungsbild der Frisur.

Eine bekannte Möglichkeit zur Milderung dieser Mißstände ist es, die Haare einer Nachbehandlung mit entsprechenden Wirkstoffen, zumeist kationischen Tensiden, die gegebenenfalls mit weiteren Substanzen kombiniert werden, zu unterziehen.

Dieses Vorgehen kann jedoch in einer Reihe von Punkten nicht befriedigen.

So ist bekannt, daß sich kationische Tenside nur für die Behandlung von nicht fettendem Haar gut eignen; ihre Anwendung bei rasch nachfettendem Haar ist dagegen problematisch, da sie die Haare zusätzlich belasten und die natürliche Nachfettung der Haare verstärken.

Weiterhin sind die üblicherweise als kationische Tenside eingesetzten quartären Ammoniumverbindungen nur unzureichend biologisch abbaubar, so daß ihr Einsatz aus ökologischen Gründen möglichst vermieden oder verringert werden sollte.

Weiterhin unbefriedigend ist, daß die Behandlung mit kationischen Tensiden in der Regel in einem separaten Schritt, üblicherweise einer Spülung, erfolgen muß, da diese kationischen Tenside sich häufig nicht in Zubereitungen mit anionischen Tensiden wie beispielsweise Shampoos einarbeiten lassen.

Eine weitere Stoffgruppe, durch deren Einsatz unerwünschte Beeinträchtigungen der Haare vermindert werden können, stellen Proteinhydrolysate dar.

Die deutsche Patentschrift DE 35 28 168 beschreibt synergistisch wirkende Mischungen aus Proteinhydrolysaten und Mono-, Di- und Oligosacchariden, die sich durch gute Filmbildungseigenschaften, wie sie für die Behandlung unerwünschter Beeinträchtigungen der Haare gewünscht werden, auszeichnen. Mischungen aus bekannten Proteinhydrolysaten und Sacchariden weisen allerdings den Nachteil auf, daß bereits während der Lagerung bei Raumtemperatur die sogenannte Maillard-Reaktion ablaufen kann. Dies führt nicht nur zum Abbau der Wirkstoffe, sondern auch zu einer Braunfärbung der Zubereitung. Diese Braunfärbung wird vom Verbraucher in der Regel nicht akzeptiert, so daß sie durch Zugabe erhöhter Mengen an Farbstoffen überdeckt werden muß. In Cosmetics & Toiletries 98, 76-84 (1983) wird als Formula 5 eine "Protein Moisturizing Lotion" offenbart, die ein quaterniertes Collagenhydrolysat, ein anionisches Polymer sowie Sorbit enthält.

Es besteht somit weiterhin ein Bedarf an haarkosmetischen Zubereitungen, die sich durch eine Verringerung der unerwünschten Beeinträchtigungen der Haare auszeichnen.

Es wurde nun überraschenderweise gefunden, daß solche haarkosmetischen Zubereitungen die Haare deutlich weniger unerwünscht beeinflussen, die ein kationisch derivatisiertes Proteinhydrolysat (A) und ein Kohlenhydrat (B) und ein kationisches, anionisches oder Ampho-Polymer (C) enthalten. Durch Einsatz dieser Wirkstoffkombinationen wird sowohl die Reißfestigkeit des Haares erhöht, als auch ein besserer Halt der Frisur und ein verbessertes Volumen erhalten. Weiterhin tritt bei den Kombinationen (A)+ (B) und (A)+-(B) + (C) keine Maillard-Reaktion auf.

Gegenstand der-Erfindung ist somit die Verwendung von Zubereitungen, enthaltend übliche Bestandteile zum Reinigen und zur Pflege der Haare sowie eine Wirkstoffkombination, bestehend aus (A) einem kationisch derivatisierten Proteinhydrolysat und (B) einem Kohlenhydrat und einem (C) kationischen, anionischen oder Ampho-Polymer, zum Reinigen und zur Pflege der Haare.

Kationisch derivatisierte Proteinhydrolysate (A) sind Substanzmischungen, die beispielsweise durch Umsetzung von alkalisch, sauer oder enzymatisch hydrolysierten Proteinen mit Glycidyltrialkylammoniumsalzen oder 3-Halo-2-hydroxypropyltrialkylammoniumsalzen erhalten werden können.

Proteine, die als Ausgangstoffe für die Proteinhydrolysate dienen, können sowohl tierischer als auch pflanzlicher Herkunft sein. Übliche Ausgangsstoffe sind beispielsweise Keratin, Kollagen, Elastin, Sojaprotein, Milchprotein, Weizenprotein, Seidenprotein und Mandelprotein.

Durch die Hydrolyse entstehen Stoffmischungen mit Molmassen im Bereich von ca. 100 bis ca. 50 000 Dalton. Übliche mittlere Molmassen liegen in einem Bereich von etwa 500 bis etwa 1000 Dalton.

Nähere Einzelheiten über kationische Derivatisierung können u.a. der japanischen Patentanmeldung 77/73485 (Chemical Abstracts Referat 90:174508v) entnommen werden.

Vorteilhafterweise enthalten die kationisch derivatisierten Proteinhydrolysate eine oder zwei lange Alkylketten mit 8 bis 22 C-Atomen und entsprechend zwei oder eine kurze Alkylkette mit 1 bis 4 C-Atomen. Verbindungen, die eine lange Alkylkette enthalten, sind bevorzugt.

Bevorzugte Verbindungen (A) sind Substanzen der Formel (I), 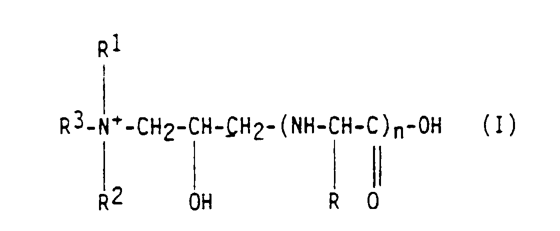 in der R für die Seitenketten der Aminosäuren des Proteins, R¹ und R² unabhängig voneinander für Alkylketten mit 1 bis 4 C-Atomen und R³ für eine Alkylkette mit 8 bis 22 C-Atomen steht.

Ein auf dem Markt erhältliches Produkt ist Lamequat^{R}L (Chemische Fabrik Grünau). Es hat die Struktur 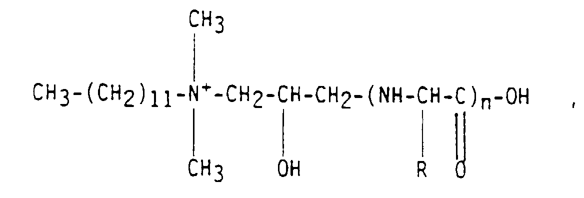 in der R für die Seitenketten der Aminosäuren des Kollagens steht. Eine Bezeichnung analog CTFA ist Lauryldimonium Hydroxypropylamino Hydrolyzed Animal Protein. Dieses Produkt stellt eine besonders bevorzugte Komponente (A) dar.

Als Kohlenhydrate (B) können erfindungsgemäß sowohl Monosaccharide als auch Oligosaccharide, wie beispielweise Rohrzucker, Milchzucker und Raffinose, eingesetzt werden. Die Verwendung von Monosacchanden ist bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose. Allose, Altrose, Glucose, Mannose, Gulose, Idose, Galactose, Talose und Fructose. Arabinose, Glucose, Galactose und Fructose sind bevorzugt eingesetzte Kohlenhydrate: Glucose ist ganz besonders bevorzugt.

Da die eingesetzten Kohlenhydrate üblicherweise aus natürlichen Rohstoffen wie Stärke gewonnen werden, weisen die Kohlenhydrate in der Regel die diesen Rohstoffen entsprechenden Konfigurationen auf (z.B. D-Glucose, D-Fructose und D-Galactose).

Beispiele für erfindungsgemäß geeignete kationische Polymere (C) sind:
- quaternierte Cellulose-Derivate, wie sie unter den Bezeichnungen Celquat^{R} und Polymer JR^{R} in Handel erhältlich sind. Die Verbindungen Celquat H 100, Celquat L 200 und Polymer JR^{R}400 sind bevorzugte quaternierte Cellulose-Derivate.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{R}734 und Gafquat^{R}755 im Handel erhältlich.
- Copolymerisate des Vinylpyrrolidons mit Vinylimidazoliummethochlorid, wie sie unter der Bezeichnung Luviquat^{R} angeboten werden.
- Polymere Dimethyldiallylammoniumsalze und deren Copolymere mit Estern und Amiden von Acrylsäure und Methacrylsäure. Die unter den Bezeichnungen Merquat^{R}100 (Poly-(dimethyldiallylammoniumchlorid)) und Merquat^{R}550 (Dimethyldiallylamnoniumchlorid-Acrylamid-Copolymer) im Handel erhältlichen Produkte sind Beispiele für solche kationischen Polymere.

Beispiele für erfindungsgemäß geeignete anionische Polymere sind:
- Polyacryl- und Polymethacrylsäuren, deren Salze, deren Copolymere mit Acrylsäure- und Methacrylsäureestern und -amiden und deren Derivate, die durch Kreuzvernetzung mit polyfunktionellen Agentien erhalten werden. Verbindungen dieser Art sind beispielsweise unter den Bezeichnungen Carbopol^{R}934, Carbopol^{R}934P, Carbopol^{R}940, Carbopol^{R}950 Carbopol^{R}980 und Hostacerin^{R}PN 73 erhältlich.
- Polyoxycarbonsäuren, wie Polyketo- und Polyaldehydocarbonsäuren und deren Salze, wie beispielsweise POC^{R}HS 5060 und POC^{R}AS5060.
- Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere. Verbindungen dieser Art sind als Luviset^{R}CA-66 und Luviset^{R}CAP erhältlich.

Unter dem Oberbegriff Ampho-Polymere sind amphotere Polymere, d.h. Polymere, die im Molekül sowohl freie Aminogruppen als auch freie -COOH- oder SO₃H-Gruppen enthalten und zur Ausbildung innerer Salze befähigt sind, zwitterionische Polymere, die im Molekül quartäre Ammoniumgruppen und-COO⁻- oder -SO₃⁻-Gruppen enthalten, und solche Polymeren zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{R} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare Ampho-Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Besonders bevorzugt eingesetzte Ampho-Polymere ist solche Polymerisate, die sich im wesentlichen zusammensetzen aus
(a) Monomeren mit quartären Ammoniumgruppen der allgemeinen Formel (II),

   R⁴-CH=CR⁵-CO-Z-(CₙH₂ₙ)-N⁽⁺⁾R⁶R⁷R⁸ A⁽⁻⁾ (II)

   in der R⁴ und R⁵ unabhängig voneinander stehen für Wasserstoff oder eine Methylgruppe und R⁶, R⁷ und R⁸ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist und
(b) monomeren Carbonsäuren der allgemeinen Formel (III),

   R⁹-CH = CR¹⁰-COOH (III)

   in denen R⁹ und R¹⁰ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R⁶, R⁷ und R⁸ Methylgruppen sind, Z eine NH-Gruppe und A⁽⁻⁾ ein Halogenid-, Methoxysulfat- oder Ethoxysulfat-lon ist; Acrylamidopropyl-trimethylammoniumchlorid ist ein besonders bevorzugtes Monomeres (a). Als Monomeres (b) für die genannten Polymerisate wird bevorzugt Acrylsäure verwendet.

Die Wirkstoffe (A) sind in einer Menge von 0,1 bis 10 Gew.-%, bezogen auf die gesamte Zubereitung, enthalten. Konzentrationen von 0.5 bis 5 Gew.% sind bevorzugt.

Die Wirkstoffe (B) sind ebenfalls in einer Menge von 0,1 bis 10 Gew.-%, bezogen auf die gesamte Zubereitung, enthalten. Konzentrationen von 0.5 bis 5 Gew.% sind bevorzugt.

Es hat sich gezeigt, daß eine besonders vorteilhafte Wirkung dann erzielt wird, wenn die Verbindungen (A) und (B) in einem Gewichtsverhältnis von 0.5:1 bis 2:1 vorliegen; Zubereitungen, in denen die Komponenten (A) und (B) in etwa gleichen Gewichtsteilen vorliegen, sind besonders bevorzugt.

Die Wirkstoffe (C) sind in den erfindungsgemäßen Zubereitungen bevorzugt in einer Menge von 0.1 bis 5, insbesondere 0,2 bis 2 Gew.-%, bezogen auf die gesamte Zubereitung, enthalten.

Wenngleich bereits die Kombinationen aus Wirkstoff (A) und Wirkstoff (B) bzw. Wirkstoff (A) und Wirkstoff (C) einen überraschend starken Effekt auf die Struktur und die Eigenschaften des Haares haben, so hat sich gezeigt, daß bei Kombination aller drei Wirkstoffe eine weitere, unerwartet große Wirkungssteigerung auftritt.

Die erfindungsgemäßen Zubereitungen können sowohl in solchen Haarbehandlungsverfahren eingesetzt werden, bei denen die Zubereitung nach Anwendung aus dem Haar wieder ausgespült werden, als auch bei solchen Haarbehandlungsverfahren, bei denen die Zubereitung im Haar verbleibt.

Zubereitungen, nach deren Anwendung die Haare üblicherweise gespült werden, sind beispielsweise Shampoos, Wellmittel und Färbemittel. Produkte, die üblicherweise im Haar verbleiben, sind beispielsweise Haarfestiger, Haarkuren und Fönwellpräparate Je nach Art der Zubereitung können neben den obligatorischen Komponenten alle für den jeweiligen Anwendungszweck üblichen Hilfs- und Zusatzmittel enthalten sein.

Bei Shampoos sind dies üblicherweiser neben der Basis Wasser anionische, nichtionische und zwitterionische Tenside, Perlglanzmittel, Parfüme, Mittel zur Einstellung des pH-Wertes, Farbstoffe, Konservierungsmittel, gegebenenfalls alkoxylierte Fettsäuren und Fettalkohole und Komponenten zur Einstellung der Viskosität.

Haarspülungen, die ebenfalls üblicherweise auf wäßriger Basis aufgebaut sind, enthalten in der Regel quartäre Ammoniumverbindungen, niedere Alkohole, Lösungsvermittler, Wirkstoffe wie z. B. Panthenol, nichtionische Polymere, Mittel zur Einstellung der Viskosität und Parfümöle.

Haarfestiger enthalten in der Regel neben Wasser niedere Alkohole, Tenside, bevorzugt nichtionogen, Stabilisatoren, kationische und/oder anionische Polymere, Mittel zur Einstellung des pH-Wertes sowie, bei Formulierung als Schaumhaarfestiger, Treibmittel.

In Haarfärbemitteln sind üblicherweiser direktziehende Farbstoffe und/oder Vorprodukte für Oxidationsfarbstoffe (Kuppler-, Entwickler-Komponenten), Tenside, Fettalkohole und Mittel zur Einstellung des pH-Wertes enthalten.

Wellmittel enthalten in der Regel neben dem Reduktionsmittel (Thioglykol-Säure, Thiomilchsäure) Komponenten zur Einstellung des pH-Wertes, Tenside. Komplexierungsmittel, Lösungsvermittler, Fettalkohole und Parfümöle. Die entsprechenden Fixierlösungen enthalten anstelle des Reduktionsmittels Oxidationsmittel wie Wasserstoffperoxid und Kaliumbromat.

### Beispiele

### 1. Bestimmung von Haareigenschaften

Es wurden 4 Shampoos getestet, deren Zusammensetzungen in Tabelle 1 aufgeführt sind.

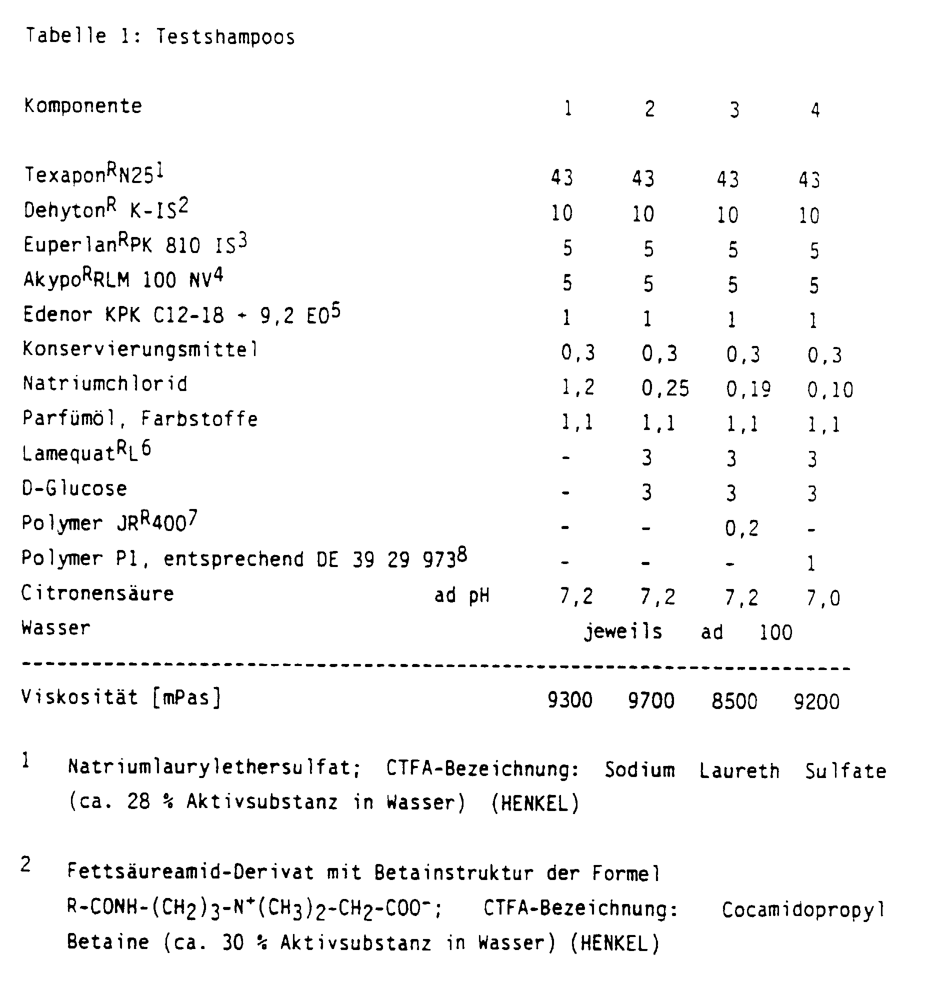

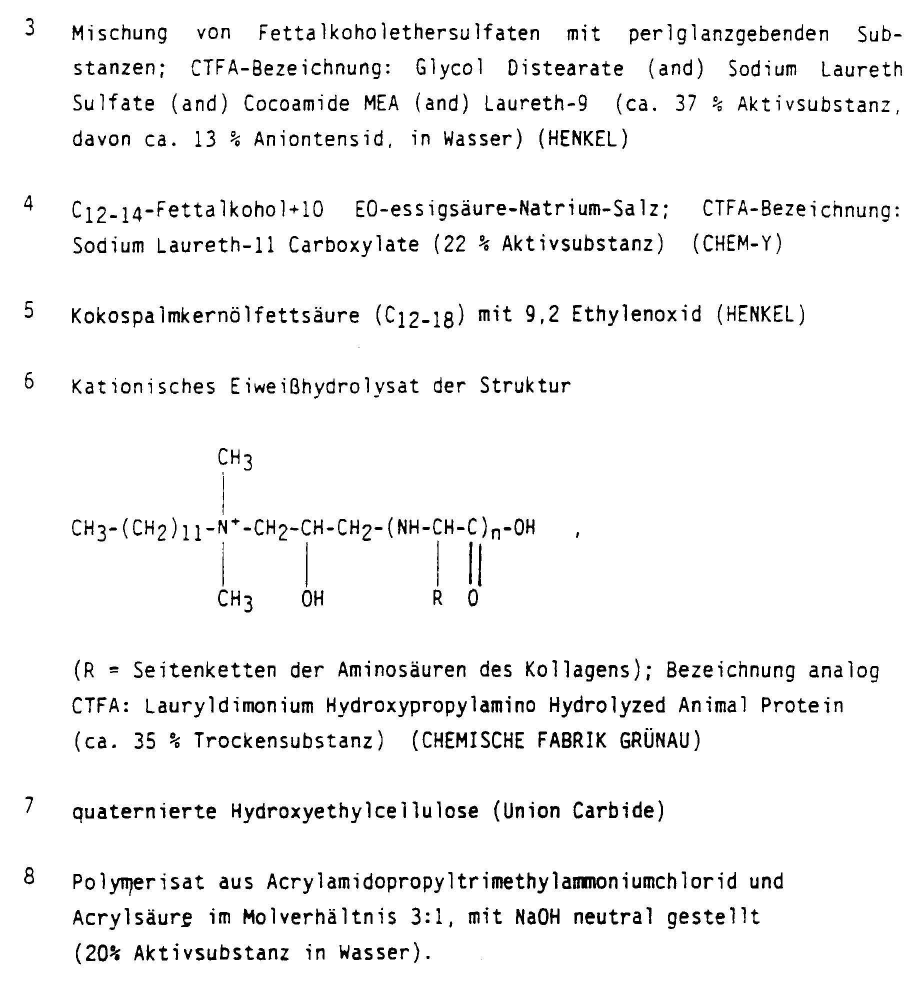

### a) Zugdehnungsmessungen an behandelten Haaren

Zu den Messungen wurden Haarsträhnen vom Typ 6621 der Firma Alkinco (Strähnenlänge: 12 cm) verwendet.

Die Haare wurden 20 Minuten lang mit dem Testshampoo behandelt, sodann ca. 30 Sekunden gespült und ca. 1,5 Stunden bei 38 °C mit einem Heizlüfter getrocknet. Anschließend wurden die Haare 20 Minuten lang ultrablondiert (Zusammensetzung der Ultrablondierung: 6 Gew.-% Wasserstoffperoxid, 15 Gew.-% Ammoniumperoxidisulfat, konz. Ammoniak ad pH 9,4, Rest Wasser) und ca. 1 Minute mit Wasser gespült. Die nassen Haare wurden wie oben ausgeführt shampooniert, gespült und getrocknet. Sodann wurden die Haare kaltgewellt (Wellösung: 7 Gew.-% Thioglykolsäure, konz. Ammoniak ad pH 9,0, Rest Wasser), ca. 1 Minute mit Wasser (38 °C) gespült und mit einer Fixierlösung (2 Gew.-% Wasserstoffperoxid, Citronensäure ad pH 4.0, Rest Wasser) behandelt, wie oben shampooniert, gespült und getrocknet. Der Zyklus Ultrablondierung, Spülung, Shampoonierung, Spülung, Trocknung, Kaltwelle, Spülung, Fixierung, Spülung, Shampoonierung, Spülung und Trocknung wurde dann noch zweimal wiederholt.
Für die Trockenbestimmung wurde das Haar bei 38 °C an der Luft temperiert; für die Naßbestimmungen wurde das Haar bis unmittelbar vor der Messung unter Wasser liegend aufbewahrt.

Es wurden folgende Größen bestimmt:
Höchstreißkraft (Zugkraft, bei der das Haar reißt).
15 %-Dehnungswert (Zugkraft, bei der das Haar um 15 % gedehnt wird),
Reißdehnung (% Dehnung, bei der das Haar reißt),
Sprödigkeit.

Einzelheiten des Meßverfahrens können der Literatur (Ärztl. Kosmetologie 15, 347 - 355 (1985) und Parfümerie & Kosmetik 72, 74 - 81 (1991)) entnommen werden.

Als Sprödigkeit wurde der Anteil der Haare bezeichnet, die bei 20% Dehnung und weniger einen Haarbruch aufwiesen.

| Naßbestimmung | Shampoo 1 | 2 | 3 | 4 |
|---|---|---|---|---|
| Höchstreißkraft [mN] | 380 | 462 | 390 | 511 |
| 15%-Dehnungswert [mN] | 268 | 307 | 290 | 304 |

| Trockenbestimmung | Shampoo 1 | 2 | 3 | 4 |
|---|---|---|---|---|
| Höchstreißkraft [mN] | 477 | 446 | 461 | 501 |
| 15%-Dehnungswert [mN] | 432 | 401 | 403 | 418 |
| Reißdehnung [%] | 21 | 28 | 28 | 42 |
| Sprödigkeit [%] | 68 | 52 | 52 | 30 |

### b) Bestimmung der Trockenkämmarbeit

Die Bestimmung der Trockenkämmarbeit erfolgte an braunem Haar (Alkinco 6634, Strähnenlänge: 12 cm; Strähnengewicht: 1 g) in Form eines Mittelwert-Paarvergleiches. Zur Bestimmung des Nullwertes wurden die Strähnen 1,5 Minuten lang mit Wasser (1 l/min, 38°C) gespült und ausgekämmt. Danach wurden die Strähnen 40 Minuten lang über einem Lüfter bei 45°C getrocknet. Nach 12stündigem Konditionieren bei 30°C und einer relativen Luftfeuchtigkeit von 40 % wurde die Kämmarbeit bestimmt. Anschließend wurden die Haarsträhnen 5 Minuten lang mit 100 g der Formulierung behandelt und dann in oben dargestellter Weise gespült, getrocknet und konditioniert. Dann wurde die Trockenkämmarbeit bestimmt. Einzelheiten des Meßverfahrens können der Literatur (Ärztl. Kosmetologie 20, 498-502 (1990)) entnommen werden.

Es wurden die folgenden Kämmarbeiten gemessen:

| Shampoo: | Trockenkämmarbeit | | v/n [%] |
|---|---|---|---|
| | vorher | nachher | |
| | [mJ] | | |
| 1 | 6,5 | 6,5 | 100 |
| 2 | 5,1 | 7,6 | 150 |
| 3 | 5,5 | 9,7 | 175 |
| 4 | 7,6 | 10,9 | 144 |

### 2. Anwendungsbeispiele

### a) Schaumhaarfestiger

| | Gew.-% |
|---|---|
| Eumulgin O5⁹ | 0,5 |
| Luviset CA-66¹⁰ | 4,5 |
| AMP-95¹¹ | 0,4 |
| Gafquat 755¹² | 0,5 |
| D-Glucose | 0,2 |
| Lamequat^{R}L | 0,2 |
| Ammoniak (25 %ige Lösung) | 0,1 |
| Parfümöl | 0,13 |
| Ethanol | 5,0 |
| Drivosol^{R}3.5¹³ | 7,0 |
| Wasser | ad 100 |

| | |
|---|---|
| ⁹ Oleylalkohol + 5 Ethylenoxid; CTFA-Bezeichnung: Oleth-5 (HENKEL) | |
| ¹⁰ Vinylacetat-Crotonsäure-Copolymer (90:10) (BASF) | |
| ¹¹ 2-Amino-2-methylpropanol (95 % Aktivsubstanz, Rest Wasser) (ANGUS CHEMIE) | |
| ¹² Dimethylaminoethylmethacrylat-Vinylpyrrolidon-Copolymer, mit Diethylsulfat quaterniert; CTFA-Bezeichnung: Polyquaternium 11 (ca. 19 % Aktivsubstanz in Wasser) (GAF) | |
| ¹³ Propan-Isobutan-Butan-Dimethylether-Gemisch (24:72:3:1) (HÜLS) | |

### b) Spülung

| | |
|---|---|
| Cremophor^{R}RH 40¹⁴ | 1.5 |
| Dehyquart^{R}SP¹⁵ | 1,0 |
| Luviskol^{R}K 30¹⁶ | 1,0 |
| Panthenol | 1,0 |
| D-Glucose | 2,0 |
| Lamequat^{R}L | 1,0 |
| Polymer P1, entsprechend DE 39 29 973 | 3,0 |
| Hydroxyethylcellulose | 1,8 |
| Ethanol (96%) | 12.0 |
| Wasser | ad 100 |

| | |
|---|---|
| ¹⁴ hydriertes Rizinusöl, mit 45 Mol Ethylenoxid pro Mol Rizinusöl umgesetzt (BASF) | |
| ¹⁵ Oxyethylalkylammoniumphosphat; CTFA-Bezeichnung: Quaternium-52 (50 % Aktivsubstanz in Wasser) (HENKEL) | |
| ¹⁶ Polyvinylpyrrolidon (95 % Aktivsubstanz, Rest Wasser) (BASF) | |

## Patentansprüche

1. Verwendung von Zubereitungen, enthaltend übliche Bestandteile zum Reinigen und zur Pflege der Haare sowie eine Wirkstoffkombination, bestehend aus
(A) einem kationisch derivatisierten Proteinhydrolysat,
(B) einem Kohlenhydrat, ausgewählt aus
- Monosacchariden,
- Oligosacchariden,
und
(C) einem kationischen, anionischen oder Ampho-Polymer,
zum Reinigen und zur Pflege der Haare.

2. Verwendung nach Anspruch 1, dadurch gekennzeichnet, daß (A) eine Verbindung der Formel (I) ist, 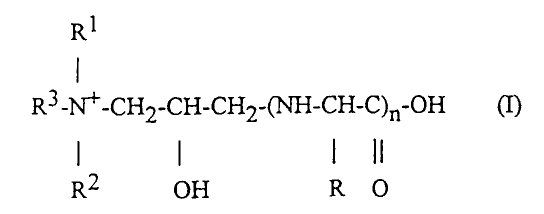 wobei R für die Seitenketten der Aminosäuren des Proteins, R¹ und R² unabhängig voneinander für Alkylketten mit 1 bis 4 C-Atomen und R³ für eine Alkylkette mit 8 bis 22 C-Atomen steht.

3. Verwendung nach Anspruch 2, dadurch gekennzeichnet, daß (A) eine Verbindung der Formel (I), in der R¹ und R² Methylgruppen und R³ eine Laurylgruppe darstellt, ist.

4. Verwendung nach einem der Ansprüche 1 bis 3, dadurch gekennzeichnet, daß als Wirkstoff (B) ein Kohlenhydrat mit 5 oder 6 Kohlenstoffatomen enthalten ist.

5. Verwendung nach Anspruch 4, dadurch gekennzeichnet, daß als Kohlenhydrat Glucose enthalten ist.

6. Verwendung nach einem der Ansprüche 1 bis 5, dadurch gekennzeichnet, daß als Wirkstoff (C) ein Ampho-Polymer enthalten ist.

7. Verwendung nach einem der Ansprüche 1 bis 6, dadurch gekennzeichnet, daß die Komponente (A) in einer Menge von 0,1 bis 10 Gew.-%, insbesondere 0,5 bis 5 Gew-%, bezogen auf die gesamte Zubereitung, enthalten ist.

8. Verwendung nach einem der Ansprüche 1 bis 7, dadurch gekennzeichnet, daß die Komponente (B) in einer Menge von 0,1 bis 10 Gew.-%, insbesondere 0,5 bis 5 Gew-%, bezogen auf die gesamte Zubereitung, enthalten ist.

9. Verwendung nach einem der Ansprüche 1 bis 8, dadurch gekennzeichnet, daß die Komponente (C) in einer Menge von 0,1 bis 5 Gew.-%, insbesondere 0,1 bis 2 Gew-%, bezogen auf die gesamte Zubereitung, enthalten ist.

10. Verfahren zur Behandlung von Haaren, dadurch gekennzeichnet, daß eine Zubereitung, wie in einem der Ansprüche 1 bis 9 verwendet, auf das Haar aufgebracht wird und nach einer Einwirkzeit wieder ausgespült wird.

11. Verfahren zur Behandlung von Haaren, dadurch gekennzeichnet, daß eine Zubereitung, wie in einem der Ansprüche 1 bis 9 verwendet, auf das Haar aufgebracht wird und dort verbleibt.

## Claims

1. The use of preparations containing typical hair-cleaning and hair-care ingredients and an active-substance combination consisting of
(A) a cationically derivatized protein hydrolyzate,
(B) a carbohydrate selected from
- monosaccharides and
- oligosaccharides
and
(C) a cationic, anionic or amphoteric polymer for the cleaning and care of the hair

2. Preparations as claimed in claim 1, characterized in that (A) is a compound corresponding to formula (I): 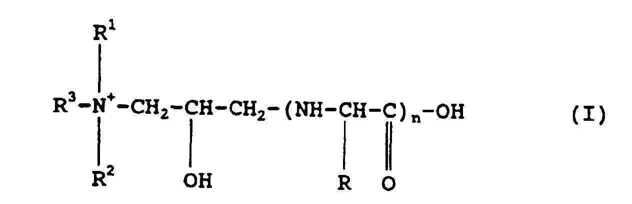 in which R represents the side chains of the aminoacids of the protein, R¹ and R² independently of one another represent alkyl chains containing 1 to 4 carbon atoms and R³ represents an alkyl chain containing 8 to 22 carbon atoms.

3. Preparations as claimed in claim 2, characterized in that (A) is a compound of formula (I) in which R¹ and R² are methyl groups and R³ is a lauryl group.

4. Preparations as claimed in any of claims 1 to 3, characterized in that a carbohydrate containing 5 or 6 carbon atoms is present as active substance (B).

5. Preparations as claimed in claim 4, characterized in that glucose is present as the carbohydrate.

6. Preparations as claimed in any of claims 1 to 5, characterized in that an amphopolymer is present as active substance (C).

7. Preparations as claimed in any of claims 1 to 6, characterized in that component (A) is present in a quantity of 0.1 to 10% by weight and, more particularly, in a quantity of 0.5 to 5% by weight, based on the preparation as a whole.

8. Preparations as claimed in any of claims 1 to 7, characterized in that component (B) is present in a quantity of 0.1 to 10% by weight and, more particularly, in a quantity of 0.5 to 5% by weight, based on the preparation as a whole.

9. Preparations as claimed in any of claims 1 to 8, characterized in that component (C) is present in a quantity of 0.1 to 5% by weight and, more particularly, in a quantity of 0.1 to 2% by weight, based on the preparation as a whole.

10. A hair treatment process, characterized in that the preparation claimed in any of claims 1 to 9 is applied to the hair and is rinsed out again after a contact time.

11. A hair treatment process, characterized in that the preparation claimed in any of claims 1 to 9 is applied to the hair and is left thereon.

## Revendications

1. Utilisation de compositions contenant des constituants usuels pour laver et soigner les cheveux, ainsi qu'une association de substances actives constituée de
(A) un hydrolysat de protéines à dérivation cationique,
(B) un hydrate de carbone, sélectionné, parmi les
- monosaccharides et les
- oligosaccharides,
et
(C) un polymère cationique, anionique ou un ampho-polymère,
pour laver et soigner les cheveux.

2. Compositions selon la revendication 1, caractérisées en ce que (A) est un composé de la formule (I), 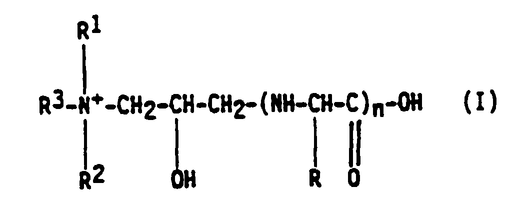 dans laquelle R représente les chaînes latérales des acides aminés de la protéine, R¹ et R² sont indépendamment l'un de l'autre des chaînes alkyle comprenant 1 à 4 atomes de C et R³ correspond à une chaîne alkyle comportant 8 à 22 atomes de C.

3. Compositions selon la revendication 2, caractérisées en ce que (A) est un composé de la formule (I), dans laquelle R¹ et R² représentent des groupes méthyle et R³ correspond à un groupe lauryle.

4. Compositions selon une des revendications 1 à 3, caractérisées en ce qu'elles renferment comme substance active (B), un hydrate de carbone comportant 5 ou 6 atomes de carbone.

5. Compositions selon la revendication 4, caractérisées en ce qu'elles renferment du glucose comme hydrate de carbone.

6. Compositions selon une des revendications 1 à 5, caractérisées en ce qu'elles renferment comme substance active (C), un ampho-polymère.

7. Compositions selon une des revendications 1 à 6, caractérisées en ce que le composant (A) est contenu dans une proportion de 0,1 à 10 % en poids, en particulier de 0,5 à 5 % en poids, par rapport à la totalité de la composition.

8. Compositions selon une des revendications 1 à 7, caractérisées en ce que le composant (B) est contenu dans une proportion de 0,1 à 10 % en poids, en particulier de 0,5 à 5 % en poids, par rapport à la totalité de la composition.

9. Compositions selon une des revendications 1 à 8, caractérisées en ce que le composant (C) est contenu dans une proportion de 0,1 à 5 % en poids, en particulier de 0,1 à 2 % en poids, par rapport à la totalité de la composition.

10. Procédé de traitement des cheveux, caractérisé en ce qu'une composition selon une des revendications 1 à 9 est appliquée sur la chevelure puis rincée après un temps d'action.

11. Procédé de traitement des cheveux, caractérisé en ce qu'une composition selon une des revendications 1 à 9 est appliquée sur la chevelure et y reste.
